# EUROPEAN PATENT SPECIFICATION

(11) **EP 0 880 965 B1**
(45) Date of publication and mention of the grant of the patent: **09.03.2005**
(21) Application number: 98108870.1
(22) Date of filing: 15.05.1998
(51) Int. Cl.: A61K 31/18, A61K 47/32, A61K 47/00

(54) **Topical pharmaceutical formulations containing nimesulide**
Nimesulid enthaltende, Topisch anwendbare Arzneipräparate
Compositions contenant de nimesulide pour application topique

(30) Priority: 26.05.1997 IT MI971226
(43) Date of publication of application: 02.12.1998
(73) Proprietor: PHARCOTERM S.p.A., 20095 Cusano Milanino (MI) (IT)
(72) Inventor: Di Schiena, Michele Giuseppe, 20080 Cisliano (MI) (IT)
(74) Representative: Gislon, Gabriele

(56) References cited:
- EP-A- 0 782 855
- NZ-A- 299 500

## Description

The present invention relates to topical pharmaceutical formulations containing nimesulide.

The systemic pharmacological treatment with non-steroidal anti-inflammatory medicaments (NSAD) is valuable in the therapy of a number of disorders characterized by phlogosis, oedema and pain, both in the human and veterinary field.

These disorders affect particularly the osteoarticular, venous, muscular systems as well as tendons, nerves, skin and mucosae.

However, the systemic therapy with NSAD frequently involves undesired, even remarkably serious, side-effects, particularly on the gastrointestinal system, in that the oral route is the one more frequently used.

In order to remove or significantly reduce such undesired side-effects, the topical therapy is preferred, whenever possible, particularly in localized areas of the human and animal body, which are easily reached by the topical pharmaceutical formulations.

Examples of said pathologies are painful and phlogistic conditions of rheumatic or traumatic nature of articulations, muscles, tendons and ligaments, as well as phlebitis, lymphangitis, superficial lymphadenitis, erythema, phlogistic and painful conditions of skin and mucosae, and the like.

Nimesulide, or N-(4-nitro-2-phenoxyphenyl)methane- sulfonamide, is a NSAD which has successfully been used in therapy for some time, and which offers the further advantage that it can be administered also to aspirin-or other NSAD- allergic patients (Goodman & Gilman's THE PHARMACOLOGICAL BASIS OF THERAPEUTICS, 9^{th} Ed.).

Nimesulide is currently used by the oral route in capsules, tablets and sachets.

Likewise the other NSAD, also nimesulide involves undesired side-effects on the gastrointestinal system; moreover it is to be used with caution in the case of elderly patients, patients with impaired renal functionality, and patients doing jobs which require quick reflexes, in that the medicament can cause sedation.

Therefore, the use of topical formulations suitable for specific pathological conditions would be useful also for the administration of nimesulide.

Up to now, however, no such topical formulations are commercially available yet, as nimesulide gives them an intense yellow colour, which is particularly evident after the application of the medicament on the skin, which results markedly yellow-stained, thus affecting the patient's compliance, which is a very important requirement for such pharmaceutical formulations.

European patent application EP 782855 and International patent application WO 96/11002 describe topical formulations of nimesulide dispersed in acrylic acid polymers. The required viscosity is obtained through the addition of a base to the acid polymer, but these documents report still a slightly yellowish colour in these topical preparations.

It has now surprisingly been found that the serious problem of the intense coloration of the formulation and of the thus treated skin can be overcome by formulating the topical pharmaceutical preparations containing nimesulide with a specific polymer excipient, which being widely used in cosmetics, is easily available, inexpensive and highly safe from the toxicologic point of view.

This peculiar polymer excipient is a sodium acrylates copolymer dispersed in oil and water by means of an emulsifier.

The emulsifier is preferably a polyether such as PPG-1 trideceth-6.

The percent composition of the polymer excipient is: polymer 40-60%, oil 30-40%, emulsifier 5-9%, water 6-10%.

The topical pharmaceutical formulations of the present invention contain the polymer excipient in concentrations ranging from 0.5% to 10%, preferably from 1% to 5%, according to the desired formulation.

Nimesulide as such or in the salified form (with physiologically acceptable organic or inorganic bases) is present in the topical pharmaceutical formulations of the present invention in concentrations from 0.1% to 5%, preferably from 0.5% to 3.5%.

The topical pharmaceutical formulations of the invention can be prepared according to procedures well known to those skilled in the art; thanks to the characteristics of the polymer excipient, it is possible to operate exclusively at room temperature, which is remarkably important for nimesulide stability.

A particularly preferred process consists in dispersing nimesulide, preferably in the micronized form, in an oil, for example squalane, adding first the polymer excipient and then, after thorough dispersion, the aqueous phase containing the stabilizers and the optional preservatives, stirring until a stable emulsion is obtained.

Other excipients and/or therapeutically complementary substances can also be added, the only restriction being their chemical-physical and therapeutical compatibility.

In the topical pharmaceutical formulations of the invention, an aqueous phase and an oily phase are present, suitably emulsified in a stable way; Examples of these formulations are thick emulsions (creams), mixed emulsions, fluid emulsions, clear emulsions etc.

The following Examples further illustrate the invention.

### EXAMPLE 1 - CREAM

Active ingredient: micronized nimesulide 3.00 g
Excipients: depurated water WAS 78.60 g; squalane (eudermal oil) 15.00 g; polymer excipient 2.50 g; lavender OE 0.50 g; polyhexanide, 2-phenoxyethanol, parabens (preservatives) 0.40 g.

The micronized nimesulide is dispersed in squalane, the polymer excipient and the lavender OE are added, mixing thoroughly at room temperature. Water containing the dissolved preservative is added with stirring. Stirring is continued vigorously for 5 minutes, then at average rate for a further 60 minutes. Stirring is stopped and the preparation is left to stand for at least 10 hours, then it is slowly mixed for 30 minutes. The cream is dispensed, for example, in tubes.

Daily dosage: repeated daily administrations to the area to treat, absorbing the cream with a gentle massage. The amount to use depends on the area to treat. The skin is not yellow-stained after the treatment.

Chemical-physical characteristics of the polymer excipient used:
INCI NAME: sodium acrylates copolymer mineral oil PPG-1 trideceth-6; Appearance at 20°C: liquid dispersion; pH stq.: 6.0-8.0; Active ingredient: 47.5-52.5%; Brookfield Viscosity (2% sol. in water): 20000-40000 cPs; Average composition: sodium acrylates copolymer 50%, mineral oil 35%, PPG-1 trideceth 6 7%, water 8%; Acute toxicity: DL₅₀ (oral, in the rat) > 2000 mg/Kg.

### EXAMPLE 2 - FLUID CREAM

The procedure of Example 1 is repeated, reducing to 1.00 g the content in polymer excipient.

This fluid cream can be used as in Example 1 and is particularly useful in the symptomatic treatment of burns with integer skin.

### EXAMPLE 3 - FLUID SPRAY CREAM

The formulation of Example 2 can be dispensed in suitable gas dispensers for the spray administration. This is particularly convenient for sport.

### EXAMPLE 4 - CREAM FOR GUMS

Active: micronized nimesulide 3.00 g.

Excipients: sterile deionized water 78.40 g; sorbitol 70% (wetting agent) 10.00 g; squalane (eudermal oil) 5.00 g; polymer excipient (as in Example 1) 2.50 g; flavour 0.50 g; polyhexanide, 2-phenoxyethanol, parabens (preservatives) 0.40 g; EDTA potassium (preservative) 0.10 g; acesulfame potassium (sweetener) 0.10 g.
The micronized nimesulide is dispersed in squalane, the polymer excipient of Example 1 and the flavour are added, mixing thoroughly. Preservatives, sorbitol and the sweetener dissolved in water are added, stirring vigorously for 5 minutes, then at average rate for a further 2 hours. The preparation is left to stand for at least 24 hours, then it is slowly mixed for 30 minutes. Use and daily dosage: massage the gum affected with phlogosis and/or pain area with about 0.5-1 cm of cream for at least 2-3 minutes; remove the excess cream without washing.

### EXAMPLE 5

The procedures of Examples 1-2-3-4 are repeated, using a polymer excipient with the following chemical-physical characteristics: INCI NAME polyquaternium 37 paraffinum liquidum PPG-1 trideceth 6; Appearance: whitish liquid; Active ingredient 48-52%; pH stq 3.2-4.8; Brookfield Viscosity (2% sol. in water) 20000-40000 cPs; Average composition Polyquaternium 37 50.0%, Paraffinum liquidum 35.0%, PPG-1 trideceth 6 7.0%, Water 8.0%; Oral acute toxicity (rat) 5000 mg/Kg.

## Claims

1. Topical pharmaceutical formulations containing nimesulide and a polymer excipient consisting of a sodium acrylates copolymer dispersed in oil and water with an emulsifier.

2. Topical pharmaceutical formulations as claimed in claim 1 wherein the polymer excipient has the following mean composition: acrylates copolymer 40-60%, oil 30-40%, emulsifier 5-9%, water 6-10%.

3. Topical pharmaceutical formulations as claimed in claims 1 or 2 wherein the emulsifier is a polyether.

4. Topical pharmaceutical formulations as claimed in claim 3 wherein the polyether is PPG-1 trideceth 6.

5. Topical pharmaceutical formulations as claimed in any one of the above claims, containing nimesulide in a corcentration from 0.1% to 5%.

6. Topical pharmaceutical formulations as claimed in any one of the above claims, containing the polymer excipient in concentrations from 0.5% to 10%

7. Process for the preparation of a topical pharmaceutical formulation according to any of claims 1 to 6, comprising the mixture of nimesulide and polymer excipient at room temperature.

8. Process according to claim 7, wherein nimesulide is first dispersed in an oil, then the polymer excipient is added and finally the aqueous phase containing the stabilisers are added to the mixture.

9. Topical pharmaceutical formulations according to claim 1 in form of a fluid spray cream containing Nimesulide 3g, squalane (15g), polymer excipient 1g, preservatives and water to 100g, and wherein the polymer excipient has the following average composition: sodium acrylates copolymer 50%, mineral oil 35%, PPG-1 trideceth 6 7% ad water 8%.

10. Topical pharmaceutical formulations according to claim 1 in form of a cream for gums containing Nimesulide 3g, sorbitol 70% 10g, squalane 5g, polymer excipient 2.50g preservatives and water to 100g, and wherein the polymer excipient has the following average composition: sodium acrylates copolymer 50%, mineral oil 35%, PPG-1 trideceth 6 7% and water 8%.

## Patentansprüche

1. Pharmazeutische Rezepturen zur lokalen Anwendung, die Nimesulid und einen Polymerträgerstoff enthalten, welcher aus Natriumacrylatcopolymer besteht, das mit einem Emulgatoren in Öl und Wasser dispergiert ist.

2. Pharmazeutische Rezepturen zur lokalen Anwendung gemäß Anspruch 1, wobei der Polymerträgerstoff folgende durchschnittliche Zusammensetzung aufweist: Acrylatcopolymer 40 - 60%, Öl 30-40%. Emulgator 5-9%, Wasser 6-10%.

3. Pharmazeutische Rezepturen zur lokalen Anwendung gemäß den Ansprüchen 1 oder 2, wobei der Emulgator ein Polyether ist.

4. Pharmaazeutische Rezepturen zur lokalen Anwendung gemäß Anspruch 3, wobei der Polyether PPG-1 Trideceth-6 ist.

5. Pharmazeutische Rezepturen zur lokalen Anwendung gemäß einem beliebigen der vorausgegangenen Ansprüche, die Nimesulid in einer Konzentration von 0,1% bis 5% enthalten.

6. Pharmazeutische Rezepturen zur lokalen Anwendung gemäß einem beliebigen der vorausgegangenen Ansprüche, die den Polymerträgerstoff in Konzentrationen von 0,5% bis 10% enfhalten.

7. Verfahren zur Herstellung einer pharmazeutischen Rezeptur zur lokclen Anwendung gemäß einem beliebigen der Ansprüche von 1 bis 6, das die Mischung von Nimesulid und dem Polymerträgerstoff bei Raumtemperatur umfasst.

8. Verfahren gemäß Anspruch 7, wobei Nimesulid zunächst in Öl dispergiert und dann der Polymerträgerstoff hinzugefügt und zum Schluss die wässrige Phase, die die Stabilisatoren enthält, zur Mischung gegeben werden.

9. Pharmazeutische Rezepturen zur lokalen Anwendung gemäß Anspruch 1 in Form einer flüssigen Spraycreme, die 3 g Nimesulid, Squalan (15 g), Polymerträgerstoff 1g, Konservierungsmittel und Wasser bis 100 g enthält, und wobei der Polymerträgerstoff die folgende durchschnittliche Zusammensetzung aufweist: Natriumacrylatcopolymer 50%, Mineralöl 35%, PPG1 Trideceth-6 7% und Wasser 8%.

10. Pharmazeutische Rezepturen zur lokalen Anwendung gemäß Anspruch 1 in Form einer Creme für Zahnfleisch, die Nimesulid 3g, Sorbitol 70% 10 g, Squalan 5g, Polymerträgerstoff 2,50g, Konservierungsmittel und Wasser bis 100g enthalten, und wobei der Polymerträgerstoff folgende durchschnittliche Zusammensetzung aufweist: Natriumacrylatcopolymer 50%, Mineralöl 35%, PPG-1 Trideceth-6 7% und Wasser 8%.

## Revendications

1. Formulations pharmaceutiques topiques contenant du nimésulide et un excipient polymère consistant en un copolymère d'acrylates de sodium dispersé dans de l'huile et dans de l'eau avec un émulseur.

2. Formulations pharmaceutiques topiques selon la revendication 1, où l'excipient polymère a la composition moyenne suivante : copolymère d'acrylates 40-60 %, huile 30-40 %, émulseur 5-9 %, eau 6-10 %.

3. Formulations pharmaceutiques topiques selon la revendication 1 ou 2, ou l'émulseur est un polyéther.

4. Formulations pharmaceutiques topiques selon la revendication 3, où le polyéther est du trideceth 6 PPG-1.

5. Formulations pharmaceutiques topiques selon l'une quelconque des revendications précédentes, contenant du nimésulide dans une cor centration allant de 0,1 % à 5 %.

6. Formulations pharmaceutiques topiques selon l'une quelconque des revendications précédentes, contenant l'excipient polymère dans des concentrations allant de 0,5 % à 10 %.

7. Procédé de préparation d'une formulation pharmaceutique topique selon l'une quelconque des revendications de 1 à 6, comprenant mélanger le nimésulide et l'excipient polymère à température ambiante.

8. Procédé selon la revendication 7, où le nimésulide est en premier dispersé dans une huile, puis l'excipient polymère est ajouté et enfin la phase aqueuse contenant les stabilisateurs est ajoutée au mélange.

9. Formulations pharmaceutiques topiques selon la revendication 1 sous forme d'une crème fluide en spray contenant du Nimésulide 3 gr, du squalane (15 gr), de l'excipient polymère 1 gr, des agents de conservation et de l'eau à 100 gr, et où l'excipient polymère a la composition moyenne suivante : copolymère d'acrylates de sodium 50 % huile minérale 35 %, trideceth 6 PPG-1 7 % et eau 8 %.

10. Formulations pharmaceutiques topiques selon la revendication 1 sous forme d'une crème pour gommes contenant du Nimésulide 3 gr, du sorbitol 70 % 10 gr, du squalane 5 gr, de l'excipient polymère 2,50 gr, ces agents de conservation et de l'eau à 100 gr, et où l'excipient polymère a la composition moyenne suivante: copolymère d'acrylates de sodium 50 %, huile minérale 35 %, trideceth 6 PPG-1 7 % et eau 8 %.
